# EUROPEAN PATENT APPLICATION

(11) **EP 2 752 198 A1**
(43) Date of publication of application: **09.07.2014**
(21) Application number: 12827124.4
(22) Date of filing: 30.08.2012
(51) Int. Cl.: A61K 39/00, A61K 39/12, A61K 45/00, A61K 47/36, A61K 47/48, A61P 31/12, A61P 35/00, C07K 14/00, C07K 19/00, C12N 15/117

(54) **VACCINE PREPARATION FOR CANCER TREATMENT**

(30) Priority: 31.08.2011 JP 2011189234
(71) Applicant: Mie University, Tsu-shi, Mie 514-8507 (JP); National University Corporation Tokyo Medical and Dental University, Bunkyo-ku Tokyo 113-8510 (JP)
(72) Inventor: SHIKU, Hiroshi, Tsu-shi Mie 514-8507 (JP); HARADA, Naozumi, Tokyo 103-0025 (JP); MURAOKA, Daisuke, Tokyo 103-0025 (JP); AKIYOSHI, Kazunari, Kyoto-shi Kyoto 615-8063 (JP)
(74) Representative: Sutcliffe, Nicholas Robert
(86) International application number: PCT/JP2012/071979
(87) International publication number: WO 2013/031882

(57) **Abstract**

[Problem] To provide a method of clearly deriving killer T cells and helper T cells in a cancer treatment vaccine in which the vaccine antibody is a synthetic long peptide which is derived from a tumor-specific antigen protein and/or pathogen-derived protein and which simultaneously contains a CD8-positive, cytotoxic T-cell recognizing epitope and a CD4-poritive, helper T-cell recognizing epitope peptide.

[Solution] BY simultaneously dosing an immunopotentiating agent with a conjugate of a hydrophobized polysaccharide (the antigen delivery system) to the synthetic long peptide, it is possible to achieve clear derivation of antigen-specific killer T-cells and helper T-cells from the cancer treatment vaccine in which the synthetic long peptide is the antigen.

## Description

### Technical Field

The present invention relates to a cancer therapy, particularly a vaccine for cancer treatment. More specifically, the invention relates to a vaccine preparation for cancer treatment comprising a synthetic long peptide as a vaccine antibody, a hydrophobized polysaccharide as an antigen delivery system, and an immunopotentiating agent which stimulates an antigen-presenting cell.

### Background Art

Results of long years of study about immunological responses against cancers revealed the importance of cellular immunity in tumor rejection of a cancer host. Particularly, it was revealed that CD8-positive cytotoxic T cells (hereinafter called killer T cells) are effector cells having effects to directly disrupt tumors, CD4-positive helper T cells (hereinafter called helper T cells) are important regulatory cells which enhance actions of the killer T cells and antigen-presenting cells, and the antigen-presenting cells typified by dendritic cells present an antigen to the T cells and stimulate it, and activate the T cells through various costimulatory molecules, cytokine or the like. As described below, rolls and positioning of each cell responsible for cellular immunological responses to tumors have been established (Non Patent Document 1).

A protein derived from a tumor cell and a protein administered as a vaccine antigen are incorporated in an antigen-presenting cell such as a dendritic cell and then cut into various lengths of peptides by proteases in the cell. In the resulting peptides, peptides having 8-10 amino acids, as antigenic epitope peptides, are loaded on a major histocompatibility complex (MHC) class I molecule and can be presented on the cell surface. The killer T cell specifically recognizes this MHC class I/antigenic peptide complex and activates it by using a T cell receptor (TCR). The activated killer T cell detects the MHC class I/antigenic peptide complex present also on the tumor cell and disrupts the tumor cell by using effector molecules such as granzyme and perforin.

Actions of helper T cells are significantly important for sufficiently activating killer T cells (Non Patent Document 2). Although an antigenic protein incorporated in an antigen-presenting cell such as a dendritic cell is cut into various lengths by the proteases in the cell, antigenic peptides having 15-20 amino acids of them form a complex with the MHC class II molecule and are presented on the antigen-presenting cell. The helper T cell specifically recognizes this complex and activates it. The activated helper T cell enhances proliferation, survival and function of the killer T cell through secretion of cytokines such as interferon (IFN)-γ and interleukin (IL)-2. Also, the helper T cell has an action to activate the dendritic cell through a CD40 ligand/CD40 pathway, and in the dendritic cell activated by the helper T cell, a stimulatory capability for the killer T cell is improved (Non Patent Document 3). It has been well known in the art that helper T cells also have an action to enhance production of antigen-specific IgG antibodies of B cells.

Thus, the killer T cell is activated by an antigen-presenting cell such as the dendritic cell in an antigen-specific manner, and the helper T cell behaves as an important enhancer which further enhances the actions of both the killer T cell and the dendritic cell. All of these three types of immunocytes are essential for effective activation of the cellular immunity to tumors. The inventors reported that the vaccine which stimulates only the killer T cells but does not activate the helper T cells delivers only inferior killer T cells and shows no therapeutic effect (Non Patent Document 4), and thus an important task is how to activate and effectively function these three types of immunocytes in development of the vaccine for cancer treatment.

In the past, the inventors prepared a polyvalent vaccine for cancer treatment of which the antigen is a recombinant full-length protein of a tumor antigenic protein in order to simultaneously activate the killer T cell and the helper T cell. The full-length protein comprises various antigenic peptides which are recognized respectively by the killer T cell and the helper T cell, and is expected to simultaneously activate the both T cells. However, in an exogenous (extracellular) antigenic protein, activation of the helper T cell through the MHC class II pathway is likely to progress, but activation of the killer T cell through the MHC class I pathway hardly progresses. This results from incorporation of the exogenous antigenic protein and mechanism of processing in an antigen-presenting cell (Non Patent Document 5).

Thus, in Japan and other countries, many trials have been made, in which an epitope peptide having 8-10 residues recognized by a short-chain peptide, mainly a killer T cell, is chemically synthesized and clinically applied as a vaccine. Since the short-chain peptide directly bonds to the MHC molecule on the cell surface without incorporation into the antigen-presenting cell and processing, its presentation to the T cell is likely to occur. In addition, production and purification of the recombinant full-length protein requires use of E. coli, mammalian cells or the like, and construction of a manufacturing system and quality control require a great deal of time and effort. In contrast, the short-chain peptide has advantages that it can be manufactured by chemical synthesis, and can be more easily manufactured than the recombinant protein.

However, some significant problems have been pointed out on the vaccine for cancer treatment comprising the short-chain peptide. Since many kinds of short-chain peptides comprise only killer T-cell recognizing epitope peptides, it is a monovalent vaccine which is not accompanied by activation of the helper T cell, in which quality of the delivered cellular immunity and therapeutic effects are insufficient (Non Patent Document 4). If the vaccine is prepared by synthesizing epitopes recognizing a killer T cell and a helper T cell as short-chain peptides respectively and composed of their mixture, delivery of a high quality killer T cell and therapeutic effects may be achieved by simultaneous activation of the killer T cell and the helper T cell. However, in this case, the epitope peptides recognizing the killer T cell and the helper T cell are administered as individual components, and therefore different dendritic cells present respective peptides, and the killer T cell and the helper T cell are unlikely to interact with each other (Non Patent Document 3).

Also, problems have been pointed out with the fact that the short-chain peptide directly bonds to the MHC molecule on the cell surface without incorporation into the antigen-presenting cell and processing (Non Patent Document 6). That is, the exogenous antigenic protein is phagocytosed by a professional antigen-presenting cell which has costimulatory molecules like a dendritic cell and a macrophage, processed in the cell, and the antigen is presented to the T cell at an adequate concentration in a manner with costimulation, whereas the short-chain peptide directly bonds to the MHC molecule on the cell surface without such steps, and thus the objective peptide is presented in a large amount in an inadequate manner even in an ordinary somatic cell which has poor abilities to incorporate (phagocytosis) and process the antigen and expresses no costimulatory molecule, resulting in the possibility of immune tolerance.

In light of the problem of vaccine comprising such short-chain peptides, usability of a long-chain synthetic peptide (long peptide) which involves respective advantages of a full-length protein capable of simultaneously activating killer T cells and helper T cells and of a cheap short-chain peptide being easily manufactured has been proposed recently (Non Patent Document 6). The long peptide is typically a polypeptide having dozens of residues which comprise at least one of each of killer T-cell recognizing epitope peptides and helper T-cell recognizing epitope peptides. It is expected that the killer T cells and the helper T cells can be simultaneously activated by the antigen-presenting cell incorporating the long peptide. In addition, the vaccine has an advantage that its manufacture is relatively easy like the short-chain peptide, because a chemical synthesis method can be used for a vaccine of which the antigen is the long peptide.

Furthermore, unlike the short-chain peptide, the long peptide, cannot directly bond to an MHC molecule as it is. The long peptide is incorporated in the dendritic cell or the like and processed in the cell, therethrough the epitope peptide recognizing the killer T cell and the helper T cell in the long peptide forms a complex with MHC molecules and is presented to the T cell at an adequate concentration in a proper manner. Additionally, in an ordinary somatic cell lacking the ability to incorporate and process antigens, the long peptide does not function as a vaccine antigen, hence the antigen is not inadequately presented to the T cell.

As seen from above, the vaccine for cancer treatment of which the vaccine antigen is the synthetic long peptide is expected to be an excellent cancer medicine which can simultaneously activate the killer T cell and helper T cell while being manufactured at a relatively low cost. However, even in the case of the vaccine for cancer treatment of which the vaccine antigen is the synthetic long peptide, when it is administered alone, the T cell cannot be sufficiently activated, and therefore efficient activation of the antigen-presenting cell such as a dendritic cell is required for maximizing the effects of the vaccine.

Immunopotentiating agents (adjuvant) have been conventionally used in order to enhance immunogenicity of a vaccine. As the immunopotentiating agent, various substances derived from bacterial cells and viruses, for example, nucleic acids (DNA and RNA), proteins, lipopolysaccharides or the like have been used, and recent research has revealed that these substances bond to Toll-like receptors on a cell surface and an intracellular site required for activation of dendritic cells to increase expressions of a costimulatory molecule (CD80 and CD86) and an MHC molecule and remarkably improve a stimulatory activity to the T cell through production of interferons and cytokines or the like. In addition to the agonist to the Toll-like receptor, it has been reported that a chemotherapeutic agent such as a taxane-based compound has an effect to activate the dendritic cell (Non Patent Document 7), and that a signaling inhibitor having an effect to prevent the dendritic cell from gaining an immunosuppressive activity can also be effective to enhance vaccine effects (Non Patent Document 8), and thus such substances may be utilized as the immunopotentiating agent.

Additionally, the inventors advanced studies on vaccine antigen delivery systems which enhance activation of the killer T cell through an MHC class I pathway of an exogenous antigenic protein in an antigen-presenting cell such as a dendritic cell or the like (called cross-presentation or cross-priming) and found their functions in a hydrophobized polysaccharide (Patent Document 1). For example, when a vaccine preparation in which an HER2 full-length protein as a tumor antigenic protein was complexed with a kind of hydrophobized polysaccharide, cholesteryl pullulan (abbr.: CHP) or cholesteryl mannan (abbr.: CHM) is administered to a human dendritic cell in vitro, it efficiently activated not only helper T cells but also killer T cells, and when it is administered to a cancer-bearing model mouse, antitumor effects were superior to those in the case where the HER2 full-length protein was administered alone (Non Patent Document 9). Similar results are reproduced also in a case using other tumor antigenic protein, NY-ESO-1 full-length protein (Non Patent Document 10). However, usabilities in low-molecular long peptides have not been clarified.

In addition, it is becoming clear that a size and a surface charge of the vaccine antigen delivery system are directly associated with a performance of the vaccine and thus important. At present, in relation to liposome as a widely-used vaccine antigen delivery system, there are reports that the larger the size is or the higher the charge is, the better the activation of the T cell and delivery of the antibody production by the vaccine are (Non Patent Documents 14-16). However, in relation to the hydrophobized polysaccharide as the vaccine antigen delivery system, such conditions have not been clarified.

### Citation List

### Patent Documents

Patent Document 1: Japanese Patent No. 4033497
Patent Document 2: Japanese Published Unexamined Patent Application No. S61-69801
Patent Document 3: Japanese Published Unexamined Patent Application No. H3-292301
Patent Document 4: Japanese Published Unexamined Patent Application No. H7-97333

### Non Patent Documents

Non Patent Document 1: Ribas, A. et al. J. Clin. Oncol. 2003; 21(12): 2415-2432.
Non Patent Document 2: Shiku, H. Int. J. Hematol. 2003; 77(5): 435-8.
Non Patent Document 3: Behrens, G. et al. Immunol. Cell Biol. 2004; 82(1): 84-90.
Non Patent Document 4: Muraoka, D., et al. J. Immunol. 2010; 185(6): 3768-76.
Non Patent Document 5: Shen L. & Rock K. L. Curr. Opin. Immunol. 2006; 18(1): 85-91.
Non Patent Document 6: Melief, C. J. M., & van der Burg, S. H. Nature Rev. Cancer, 2008; 8(5): 351-360.
Non Patent Document 7: Byrd-Leifer, C. A., et al. Eur. J. Immunol. 2001; 31(8): 2448-57.
Non Patent Document 8: Kong, L. Y., et al. Clin. Cancer Res. 2008; 14(18): 5759-68.
Non Patent Document 9: Gu, X. G., et al. Cancer Res., 1998; 58 (15) : 3385-90.
Non Patent Document 10: Hasegawa, K., et al. Clin. Cancer Res., 2006; 12(6): 1921-27.
Non Patent Document 11: Akiyoshi, K., et al. Macromolecules., 1993; 26(12): 3062-68.
Non Patent Document 12: Akiyoshi, K., et al. J. Proc. Japan. Acad., 1995; 71(71B): 15.
Non Patent Document 13: Nishikawa, T., et al. Macromolecules. 1994; 27(26): 7654-59.
Non Patent Document 14: Brewer, J. M. , et al . J. Immunol. 1998; 161: 4000-7.
Non Patent Document 15: Henriksen-Lacey, M., et al. J. Controlled Release. 2011; 154(2): 131-7.
Non Patent Document 16: Nakanishi, T., et al. J. Controlled Release. 1999; 61: 233-40.

### Summary of the Invention

### Problems to be Solved by the Invention, and Means for Solving the Problems

The inventors prepared a long peptide which is derived from a tumor-specific antigenic protein and/or pathogen-derived antigenic protein and simultaneously contains epitopes recognizing killer T cells and helper T cells, and studied a method for maximizing therapeutic effects on cancers. As a result, after keen examination, the inventors found that remarkable activation of the killer T cells and helper T cells could be delivered in a mouse model, only after combining an immunopotentiating agent with a conjugate of a hydrophobized polysaccharide CHP as an antigen delivery system to the synthetic long peptide, and completed the present invention basically.

Thus, the vaccine preparation for cancer treatment related to the present invention for solving the problems is characteristically composed of: a drug which comprises a complex of one or more synthetic long peptides derived from a tumor-specific antigenic protein and/or pathogen-derived antigenic protein and simultaneously containing a CD8 positive, cytotoxic T-cell recognizing epitope and a CD4 positive, helper T-cell recognizing epitope with a hydrophobized polysaccharide; and one or more immunopotentiating agents.

At this time, the synthetic long peptide is preferably a polypeptide composed of 23-120 amino acids comprising at least two or more T-cell recognizing epitopes. Also, the synthetic long peptide is preferably a polypeptide composed of 23-80 amino acids comprising at least two or more T-cell recognizing epitopes. Also, the synthetic long peptide is preferably a polypeptide composed of 23-40 amino acids comprising at least two or more T-cell recognizing epitopes.

Also, the CD8 positive, cytotoxic T-cell recognizing epitope is preferably a part of the amino acid sequence of the tumor-specific antigenic protein.

Also, the CD4 positive, helper T-cell recognizing epitope is preferably a part of the amino acid sequence of the tumor-specific antigenic protein.

At this time, the tumor-specific antigenic protein is preferably selected from MAGE family or NY-ESO-1, SPANX family, HER2, WT1, hTERT, RHAMM and survivin.

Also, the CD8-positive helper T-cell recognizing epitope is preferably a part of the amino acid sequence of the pathogen-derived protein.

Also, the CD4 positive, helper T-cell recognizing epitope is preferably a part of the amino acid sequence of the pathogen-derived protein.

At this time, the pathogen-derived antigenic protein is preferably selected from hepatitis B virus, hepatitis C virus, EB virus, human papillomavirus, human T-lymphotropic virus, human herpes virus 8 and human immunodeficiency virus.

Also, the polysaccharide constituting the hydrophobized polysaccharide is preferably a pullulan or a mannan.

Also, a hydrophobic group of the hydrophobized polysaccharide is preferably a cholesterol.

Also, the hydrophobized polysaccharide is preferably non-ionic.

Also, a particle size after complexation of the hydrophobized polysaccharide and the synthetic long peptide is preferably 180 nm or less. Also, the particle size after complexation of the hydrophobized polysaccharide and the synthetic long peptide is more preferably 100 nm or less.

Also, the immunopotentiating agent is preferably an agonist of a Toll-like receptor. At this time, the agonist of the Toll-like receptor is preferably a poly-IC RNA or a CpG oligo DNA.

Also, the immunopotentiating agent is preferably a chemotherapeutic agent which activates antigen-presenting cells. At this time, the chemotherapeutic agent activating the antigen-presenting cells is preferably a taxane-based compound or an anthracycline-based compound.

Also, the immunopotentiating agent is preferably an agent having effects to prevent the antigen-presenting cell from gaining an immunosuppressive activity. At this time, the agent having effects to prevent the antigen-presenting cell from gaining an immunosuppressive activity is preferably a JAK/STAT inhibitor or an IDO inhibitor.

In the present invention, the long peptide is a polypeptide characteristically comprising at least two or more T-cell recognizing epitopes included in a tumor-specific antigenic protein and/or pathogen-derived antigenic protein. The T-cell recognizing epitope may be any epitope included in the tumor-specific antigenic protein or pathogen-derived antigenic protein, and can be selected from: T-cell recognizing epitopes included in the tumor-specific antigenic protein such as MAGE family molecules like MAGE-A1, MAGE-A2, MAGE-A3, MAGE-A4, MAGE-A5, MAGE-A6, MAGE-A8, MAGE-A9, MAGE-A10, MAGE-A11, MAGE-A12, MAGE-B1 and MAGE-B2; NY-ESO-1 molecules; LAGE; SAGE family molecules; XAGE family molecules; SPANX family molecules; HER2; WT1; hTERT; RHAMM; and survivin: and T-cell recognizing epitopes included in the pathogen-derived antigenic protein such as hepatitis B virus (HBV), hepatitis C virus (HCV), EB virus, human papillomavirus (besides type 16 and type 18, types 31, 33, 35, 39, 45, 51, 52, 56, 58, 59, 68, 69, 73 and 82 can be selected), human T-lymphotropic virus (HTLV-1), human herpes virus 8 and human immunodeficiency virus. The T-cell recognizing epitope includes a CTL epitope recognized by the killer T cell and a Th epitope recognized by the helper T cell. Although it is preferable that the long peptide of the present invention simultaneously contains at least one of each of the CTL epitope and the Th epitope, the long peptide comprising the CTL epitope and the long peptide comprising the Th epitope can be used alone or in combination.

When a length of the long peptide is 22 amino acids or less, the effects of the invention cannot be generated because of immune tolerance by the direct bond to the MHC molecule, and thus such a length is undesirable. Also, when the length is 120 amino acids or longer, the effects of the invention cannot be sufficiently obtained, and thus such a length is also undesirable. Specifically, 23-120 amino acids are preferable, 23-80 amino acids are more preferable, and 23-40 amino acids are even more preferable. Two or more of the long peptides may be combined for use.

The hydrophobized polysaccharide used in the present invention can be manufactured, for example, by well-known per se methods described in Non Patent Documents 11-12, Patent Documents 2-4 and the like.

The polysaccharide in the hydrophobized polysaccharide is not particularly limited, if the sugar residue is a glycosidically-bound polymer. As a sugar residue constituting the polysaccharide, for example, a residue derived from a saccharide such as a monosaccharide like glucose, mannose, galactose and fucose, or a disaccharide or an oligosaccharide can be used. The sugar residue may be 1,2-, 1,3-, 1,4- or 1,6-glycosidically-bound, and the bond may be either α- or β-bond. In addition, the polysaccharide may be either linear or branched. As the sugar residue, a glucose residue is preferable, and as the polysaccharide, natural or synthetic pullulan, dextran, amylose, amylopectin or mannan, preferably mannan, pullulan or the like is used. A polysaccharide having an average molecular weight of 50,000-150,000 can be used.

In the hydrophobic group, for example, preferably 1 to 5 single and double-strand alkyl groups or sterol residues are introduced per 100 monosaccharides (5% or less in weight ratio), and more preferably 1 to 3 groups are introduced per 100 monosaccharides (3% or less in weight ratio). Note that the hydrophobic group is not limited to the above-described groups, and a group having a high ratio of inclusion can arbitrarily be selected according to a molecular weight and an isoelectric point of an antigen to be included. The sterol residue may include, for example, residues of cholesterol, stigmasterol, β-sitosterol, lanosterol, ergosterol or the like, preferably the cholesterol residue is used. In addition, as the alkyl group, an alkyl group having preferably less than 20 carbon atoms, more preferably 10-18 carbon atoms can be used, and they may be either linear or branched.

As the hydrophobized polysaccharide, for example, a polysaccharide in which a primary hydroxyl group having 1-5 sugar units per 100 sugar units constituting the polysaccharide is represented by the following formula (I): -O-(CH₂)mCONH(CH₂)nNH-CO-O-R (I) (wherein R is an alkyl group or a sterol residue; m is 0 or 1; n is any positive integer) is preferable. Here, the above-described groups can be preferably used as the alkyl group or the sterol residue, wherein n is preferably 1 to 8.

Note that the hydrophobized polysaccharide may be a polysaccharide bonded through the linker as described in Patent Document 4.

Also, the hydrophobized polysaccharide is preferably non-ionic, and a zeta potential under a physiological condition is preferably -2.0 mV to +2.0 mV after complexation with the long peptide. The particle size under the physiological condition is preferably less than 180 nm, more preferably 100 nm or less after complexation with the long peptide.

The complex of the long peptide and the hydrophobized polysaccharide can be purified by various chromatography techniques after the long peptide and aggregated microparticles of the hydrophobized polysaccharide are mixed at room temperature or a low temperature (Non Patent Document 13). Although the resulting complex of the long peptide and the hydrophobized polysaccharide can be directly used as the vaccine preparation of the present invention, some manipulations such as sterilization can be added according to a conventional method, as required.

The immunopotentiating agent may be any agent having effects to enhance activities of the antigen-presenting cell, more specifically, a Toll-like receptor agonist, other antigen-presenting cell-stimulating agent, and an agent having effects to prevent the antigen-presenting cell from gaining an immunosuppressive activity are selected. The Toll-like receptor agonist can be selected from an inactivated bacterial cell and a bacterial extract, a nucleic acid, a lipopolysaccharide, a lipopeptide, a synthetic low-molecular compound and the like, and preferably, CpG oligo DNA, poly-IC RNA, monophosphoryl lipid, lipopeptide, or the like is used. For the antigen-presenting cell-stimulating agent, a taxane-based agent or an anthracycline-based agent can be used. The agent having the effects to prevent the antigen-presenting cell from gaining the immunosuppressive activity is selected from a JAK/STAT inhibitor, an indole deoxygenase (IDO) inhibitor, a tryptophane deoxygenase (TDO) inhibitor and the like. This inhibitor includes compounds having an antagonism to the factor, as well as a neutralizing antibody for the factor, a small interfering RNA (siRNA) and an antisense DNA.

Although the vaccine for cancer treatment of the present invention can be administered by any method, it is preferably administered by an adequate parenteral route, for example, intravenous, interperitoneal, subcutaneous, intracutaneous, intra-adipose and intramammary routes; inhalation; intramuscular injection; or a method through a mucosal route in the form of a nasal drop or the like; etc.

The vaccine for cancer treatment of the present invention can generally be a preparation in the form suitable for subcutaneous, intravenous and intramuscular parenteral administration, as a kit in which the active ingredient, i.e. the complex of the synthetic long peptide and the hydrophobized polysaccharide and the immunopotentiating agent component are separately prepared. A dose of the vaccine preparation for cancer treatment of the present invention required for delivering the objective immunity can arbitrarily be decided, and for the normal dose, e.g. an amount of about 0.1 mg to 5 mg/dose of synthetic long peptide is administered as an indication. It is important that the active ingredients of both preparations substantially coexist simultaneously at a site of administration, and therefore both preparations are desirably administered substantially at the same time or successively. It is appropriate that number of doses is 2 to 20. Although the interval of administrations is selected from 1 to 12 weeks, a longer interval may be selected at a later phase of the treatment.

### Effects of the Invention

According to the present invention, the vaccine for cancer treatment having remarkably high therapeutic effects on cancer can be provided.

### Brief Description of the Drawings

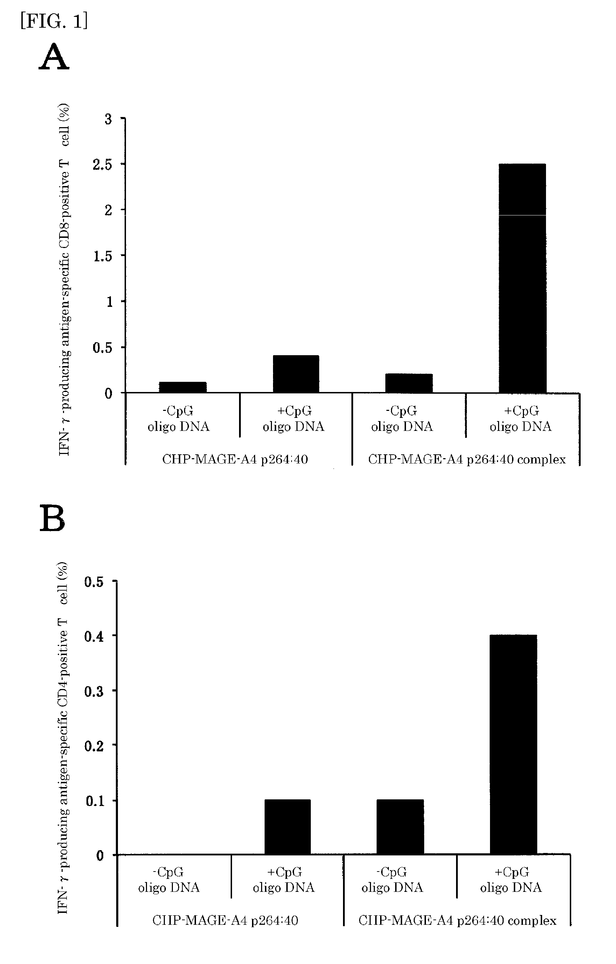

Figs. 1 are graphs of an experiment in which a complex of a synthetic long peptide prepared, having 40 amino acids, derived from an amino acid sequence of tumor-specific protein MAGE-A4 (MAGE-A4, p264:40) and a kind of hydrophobized polysaccharides, CHP (wherein 1.7 cholesteryl groups were introduced into a pullulan per 100 monosaccharides. Average molecular weight: 100,000) (CHP-MAGE-A4, p264:40 complex). The MAGE-A4 p264:40 or the CHP-MAGE-A4 p264:40 complex was subcutaneously administered simultaneously with CpG oligo DNA as an immunopotentiating agent (twice, at one week intervals) to mice. One week after the final administration, spleen cells were collected to measure percentages of CD8-positive killer T cells (Fig. 1A) or CD4-positive helper T cells (Fig. 1B) specific to an epitope contained in the synthetic long peptide by an intracellular cytokine staining method.

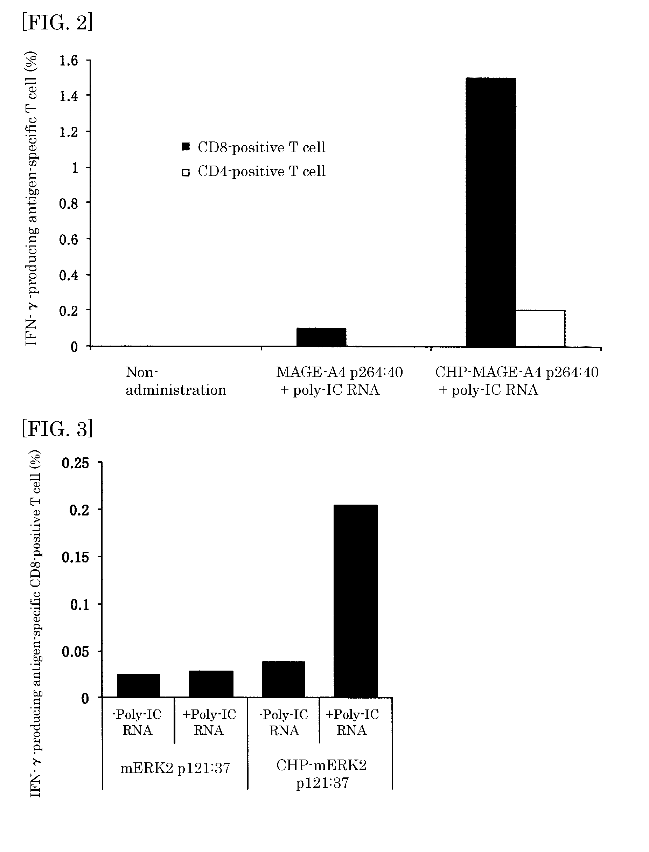

Fig. 2 is a graph of the same experiment as in Fig. 1 which was conducted using a poly-IC RNA as the immunopotentiating agent. The black bar and the white bar represent percentages of the antigen-specific CD8-positive killer T cells and the antigen-specific CD4-positive helper T cells, respectively.
Fig. 3 is a graph of the same experiment as in Fig. 2 which was conducted using the synthetic long peptide mERK2 p121:37 of 37 aa derived from an amino acid sequence of a mutated ERK2 (mERK2) which is a mouse tumor antigen, as the synthetic long peptide. The values are percentages of the antigen-specific CD8-positive killer T cells.

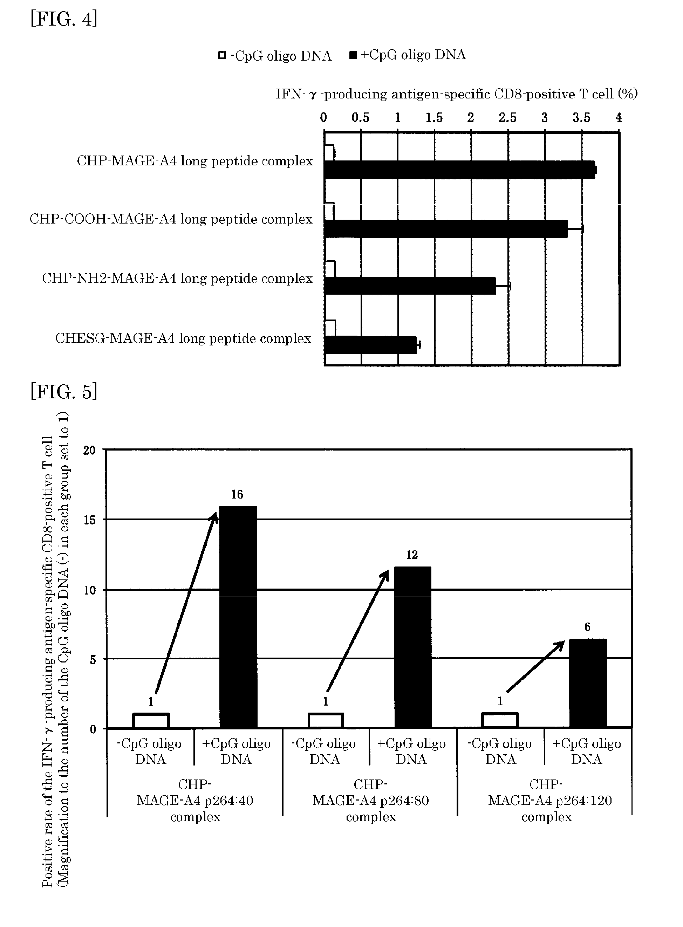

Fig. 4 is a graph of the same experiment as in Fig. 1 which was conducted using, as the hydrophobized polysaccharides, CHP, CHP-NH₂ (wherein 2 cholesteryl groups and 13 amino groups were introduced into a pullulan per 100 monosaccharides. Average molecular weight: 100,000), CHP-COOH (wherein 1.2 cholesteryl groups and 27 carboxyl groups were introduced into a pullulan per 100 monosaccharides. Average molecular weight: 100,000) and CHESG (wherein 0.8 cholesteryl groups were introduced into a glycogen per 100 monosaccharides. Average molecular weight: 2,100,000) were prepared, each of them was complexed with the synthetic long peptide MAGE-A4 p264:40. The particle sizes of each hydrophobized polysaccharide-synthetic long peptide complex and zeta potential are shown in Table 1.
Fig. 5 is a graph of the same experiment as in Fig. 1 which was conducted using the synthetic long peptide of 40 aa, 80 aa or 120 aa derived from the amino acid sequence of MAGE-A4 (MAGE-A4 p264:40, MAGE-A4 p264:80, MAGE-A4 p264:120). The p264:80 and p264:120 comprise 2 or 3 repeated sequences of p264:40, respectively.

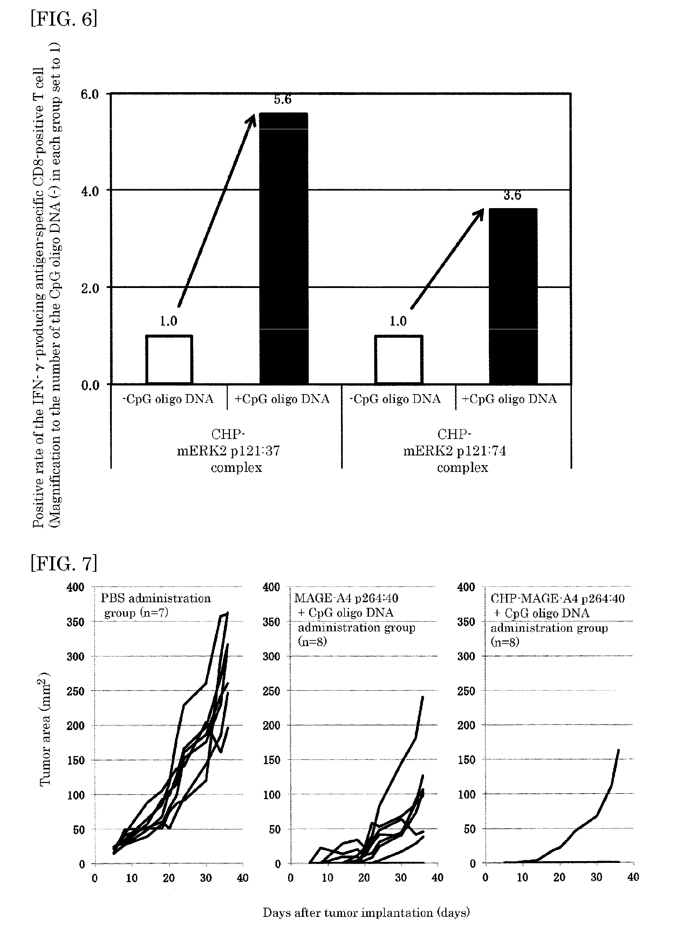

Fig. 6 is a graph of the same experiment as in Fig. 1 which was conducted using the synthetic long peptide of 37 aa or 74 aa derived from the amino acid sequence of mERK2 (mERK2 p121:37, mERK2 p121:74). The p121:74 comprises 2 repeated sequences of p121:37.
Figs. 7 are graphs of an experiment in which the MAGE-A4 p264:40 or the CHP-MAGE-A4 p264:40 complex was subcutaneously administered simultaneously with CpG oligo DNA to BALB/c mice. Seven days after the administration, mouse colon cancer cell CT 26 to which MAGE-A4 gene was stably introduced was subcutaneously implanted. Subsequent transitions of tumor areas in each individual are shown in the graph. Both the administration group of MAGE-A4 p264:40/CpG oligo DNA and the administration group of CHP-MAGE-A4 p264:40 complex/CpG oligo DNA showed significant suppression (p<0.0001) of tumor proliferation compared to an administration group of phosphate buffered saline (PBS), but the CHP-MAGE-A4 p264:40 complex/CpG oligo DNA administration group showed more remarkable antiproliferative effects.

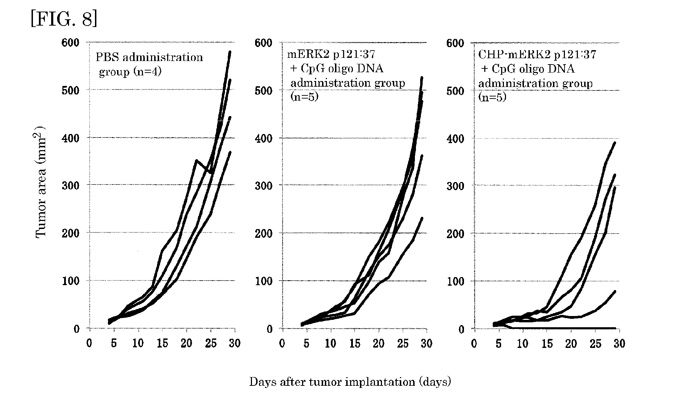

Figs. 8 are graphs of an experiment in which the mERK2 p121:37 or the CHP-mERK2 p121:37 complex was subcutaneously administered simultaneously with CpG oligo DNA to BALB/c mice. Seven days after the administration, mouse fibrosarcoma cell CMS5a which expresses mERK2 protein was subcutaneously implanted. Subsequent transitions of tumor proliferation in each individual are shown in the graph. Only the CHP-mERK2 p121:37 complex/CpG oligo DNA administration group showed significant suppression (p<0.03) of tumor proliferation compared to the PBS administration group.

### Best Mode for Carrying Out the Invention

Next, embodiments of the present invention will be explained with reference to figures and tables, but the technical scope of the present invention is not limited by these embodiments and can be carried out in various configurations without changing the gist of the invention. In addition, the technical scope of the present invention covers the scope of equivalence.

### <Material and Method>

### (1) Material

BALB/c mice (female, 5 weeks old) were purchased from Japan SLC, Inc., and reared in the Animal Center of the Faculty of Medicine of Mie University. After a week of conditioning, they were used for an experiment. An experiment design using mice received approval from the Ethics Committee of the Faculty of Medicine of Mie University. The synthetic long peptide was purchased from GenScript Inc., Scrum Inc., or Biologica Co. The sequence of the synthetic long peptide is as follows: MAGE-A4 p264:40 (amino acid sequence (SEQ ID NO: 1): GSNPARYEFLWGPRALAETSYVKVLEHVVRVNARVRIAYP, wherein the sequence of 9 amino acids from the 2th S to the 10th L (SEQ ID NO: 2: SNPARYEFL) are included as the killer T cell epitope sequence, and the sequence of 16 amino acids from the 22th V to the 37th (SEQ ID NO: 3: VKVLEHVVRVNARVRI) are included as the helper T cell epitope). MAGE-A4 p264:80 (amino acid sequence (SEQ ID NO: 4) :
GSNPARYEFLWGPRALAETSYVKVLEHVVRVNARVRIAYPGSNPARYEFLWGPRALAETSYV KVLEHVVRVNARVRIAYP, two repeated sequences of p264:40). MAGE-A4 p264:120 (amino acid sequence (SEQ ID NO: 5): GSNPARYEFLWGPRALAETSYVKVLEHVVRVNARVRIAYPGSNPARYEFLWGPRALAETSYV KVLEHVVRVNARVRIAYPGSNPARYEFLWGPRALAETSYVKVLEHVVRVNARVRIAYP, three repeated sequences of p264:40). mERK2 p121:37 (amino acid sequence (SEQ ID NO: 6): NDHIAYFLYQILRGLQYIHSANVLHRDLKPSNLLLNT, wherein the sequence of 9 amino acids from the 16th Q to the 24th L (SEQ ID NO: 7: QYIHSANVL) are included as the killer T cell epitope sequence, and the sequence of 17 amino acids from the 13th R to the 29th K (SEQ ID NO: 8: RGLQYIHSANVLHRDLK) are included as the helper T cell epitope sequence). mERK2 p121:74 (amino acid sequence (SEQ ID NO: 9): NDHIAYFLYQILRGLQYIHSANVLHRDLKPSNLLLNTNDHIAYFLYQILRGLQYIHSANVLH RDLKPSNLLLNT, two repeated sequences of p121:37). The synthetic peptide was purchased from Sigma-Genosys Ltd. The amino acid sequence of the peptide is as follows: MAGE-A4 p265 (amino acid sequence (SEQ ID NO: 2) : SNPARYEFL), mERK2 9m (amino acid sequence (SEQ ID NO: 7) : QYIHSANVL). CHP was purchased from NOF CORPORATION. In synthesis of CHP-NH₂, CHP and 1,1'-carbonyldiimidazole were reacted using DMSO as a solvent under a nitrogen atmosphere, to which ethylenediamine was then added to react it. The reaction liquid was dialyzed and purified, then lyophilized to obtain CHP-NH₂. In synthesis of CHP-COOH, CHP and a succinic anhydride were reacted using DMSO as a solvent, and N,N-dimethyl-4-aminopyridine as a catalyst under a nitrogen atmosphere. After the reaction, the product was re-deposited and dialyzed and then lyophilized to obtain CHP-COOH. In synthesis of CHESG, an enzyme-synthesized glycogen and cholesteryl isocyanate were reacted using DMSO as a solvent, and dibutyltin dilaurate as a catalyst under a nitrogen atmosphere. After the reaction, the product was re-deposited and dialyzed and then lyophilized to obtain CHESG. As the immunopotentiating agent, the poly-IC RNA was purchased from Oncovir, Inc. CpG oligo DNA was purchased from Hokkaido System Science Co., Ltd. The imiquimod was purchased from Sigma-Aldrich Co. A FITC-labeled anti-CD4 monoclonal antibody (clone RM4-5), PerCP-Cy5.5-labeled anti-CD8 monoclonal antibody (clone 53-6.7), APC-labeled anti-IFNγ antibody (clone XMG1.2) and PE-labeled anti-IL-2 antibody (clone JES6-5H4) were purchased from eBioscience, Inc., or BD Biosciences Company.

### (2) Preparation of the complex of the hydrophobized polysaccharide and the synthetic long peptide

10 mg/mL of synthetic long peptide was dissolved in dimethylsulfoxide (DMSO). 10 mg/mL of hydrophobized polysaccharide was dissolved in phosphate buffered saline (PBS) containing 6 M of urea. 20 mL (200 mg) of hydrophobized polysaccharide solution was added to 1 mL (10 mg) of a synthetic long peptide solution, and left in a dark place at room temperature overnight. It was transferred to a dialysis device (molecular weight cut-off: 3500, Thermo Fisher Scientific K.K.), and dialyzed to PBS containing 0.6 M of urea at more than a hundredfold volume ratio as a dialysate fluid at room temperature for 2 hours to overnight. Subsequently, it was dialyzed to PBS containing 0.06 M of urea at more than a hundredfold volume ratio as a dialysate fluid at room temperature for 2 hours to overnight. Again, it was dialyzed to PBS at more than a hundredfold volume ratio as a dialysate fluid at room temperature for 2 hours to overnight. A non-dialyzable fluid was collected, and UV absorption (280 nm) was measured to determine the final concentration of the synthetic long peptide. The particle size of the hydrophobized polysaccharide-synthetic long peptide complex was measured using a dynamic light scattering photometer (Zetasizer, Malvern Instruments Ltd.), and Z-Average value was adopted. The zeta potential was measured using a zeta potential measuring device (SZ-100, HORIBA, Ltd.).

### (3) Administration method

The hydrophobized polysaccharide-synthetic long peptide complex and the immunopotentiating agent were simultaneously administered to mice. For administration, they were subcutaneously injected into a mouse's back twice at one week intervals. For a dose of the hydrophobized polysaccharide-synthetic long peptide complex, a dosage corresponding to 0.1 mg of the synthetic long peptide complex was administered. Any of CpG oligo DNA or poly-IC RNA as the immunopotentiating agent was subcutaneously injected at a dose of 0. 05 mg in proximity to the administration site of the hydrophobized polysaccharide-synthetic long peptide.

### (4) Isolation and purification of mouse immunocytes

One week after the final administration, spleen cells were isolated from the dosed mice in a manner shown below. The spleen was isolated from the mouse and washed in a RPMI1640 medium to remove blood. The spleen was grinded using a slide glass, and then the free cells were collected in the RPMI1640 medium. After centrifugation (400xg, 5 min., 4°C), the supernatant was removed, to which 2 mL of ACK solution was added to process the cells for 1 minute. 18 mL of the RPMI1640 medium was added and centrifugation (400xg, 5 min., 4°C) was carried out. The supernatant was removed, and the cells were suspended in an adequate amount of the RPMI1640 medium. After the number of the cells was counted, the cells were suspended in the RPMI1640 medium containing a 10% fetal bovine serum (FBS) so that the cell concentration was 1×10⁷ cells/mL.

### (5) Intracellular cytokine staining

The isolated spleen cells were added to a 24-well culture plate (Nunc Co., Ltd.) at 5×10⁶/0.5 mL per one well. As a peptide for re-stimulation, 10 µM of MAGE-A4 p264, MAGE-A4 p265 or mERK2 9m was added and cultured under 5% CO₂ at 37 °C for 6 hours. Subsequently, BD GoldiPlug™ (BD Biosciences Company) 10-fold-diluted with the RPMI1640 medium containing 10% FBS was added at 50 µL/well, and cultured under 5% CO₂ at 37°C for 6 hours. The cells were collected and transferred to a 96 well round-bottom microplate (Nunc Co., Ltd.). After a supernatant was removed by centrifugation (1200 rpm, 1 min., 4°C), the cells were suspended in a dyeing buffer (PBS containing 0.5% bovine serum albumin) at 50 µL/well. A manufacturer-recommended amount of FITC-labeled anti-CD8 monoclonal antibody or FITC-labeled anti-CD4 monoclonal antibody was added, mixed, and then left in a dark place at 4°C for 15 minutes. After the cells were washed with 200 µL of dyeing buffer twice, 100 µL of BD Cytofix/Cytoperm™ buffer (BD Biosciences Company) was added and mixed very gently. It was left in a dark place at room temperature for 20 minutes, and then washed with 100 µL of BD Perm/Wash™ buffer (BD Biosciences Company) twice. 50 µL of BD Perm/Wash™ buffer containing various anti-cytokine antibody was added to the cells, which was gently suspended and then left in a dark place at room temperature for 15 minutes. The cells were washed with 100 µL of BD Perm/Wash™ buffer twice, then re-suspended in 200 µL of dyeing buffer, and transferred to a round-bottom polystyrene tube (BD Biosciences Company).

### (6) Flow cytometry analysis

The cells were analyzed by a flow cytometer (FACS Canto II, BD Biosciences Company) using an accompanying analysis software (FACSDiva).

### (7) Tumor rejection test

A mouse colon cancer cell CT26 cell line into which MAGE-A4 gene was stably introduced or a mouse fibrosarcoma CMS5a cell line which expresses mERK2 was subcutaneously implanted in a manner shown below. The CT26 cell line or CMS5a cell line cultured in a T75 flask (Nunc Co. , Ltd.) was washed with PBS, and then the cells were removed with PBS containing 0.5% trypsin and collected in the RPMI1640 medium containing 10% FBS. After centrifugation (400xg, 5 min., 4°C), the supernatant was removed, washed with the RPMI1640 medium twice, then re-suspended in the RPMI1640 medium at a concentration of 1×10⁶ cells/100 µL, and subcutaneously implanted to BALB/c mice in an amount of 100 µL/individual. Subsequently, their tumor areas were measured over time.

### (8) Statistical analysis

In the tumor rejection test, data were compared by Student t test using MS Excel (Microsoft Corporation).

### <Test results>

A graph of Fig. 1 shows that the specific killer T cells and helper T cells of which the antigen is the synthetic long peptide can be clearly delivered by simultaneously administering the immunopotentiating agent with a conjugate of the hydrophobized polysaccharide to the synthetic long peptide. When the synthetic long peptide of 40 amino acids (aa) derived from the tumor-specific protein MAGE-A4 (MAGE-A4 p264:40) is administered alone to mice, the killer T cells and helper T cells specific to said peptide are not delivered. When the CpG oligo DNA (Toll-like receptor 9 agonist) as the immunopotentiating agent is administered with the peptide, the objective T cells are not delivered. However, when the peptide is simultaneously administered with the CpG oligo DNA with a conjugate of the CHP which is a kind of hydrophobized polysaccharides, deliveries of the antigen-specific killer T cells and helper T cells can be clearly observed. When only the CHP-MAGE-A4 p264:40 complex is administered, such deliveries of the T-cells do not occur. These results show that the long peptide vaccine can receive maximum effects to enhance the CpG oligo DNA as the immunopotentiating agent only after being complexed with the hydrophobized polysaccharide CHP.

The poly-IC RNA (Toll-like receptor 3 agonist) was used instead of the CpG oligo DNA as the immunopotentiating agent to conduct the same test as in Fig. 1. As anticipated, the effects of the immunopotentiating agent were more remarkable in the CHP-synthetic long peptide complex than in the synthetic long peptide alone (Fig. 2). These results suggest that as long as the immunopotentiating agent which enhances immune delivery by administration of the CHP-synthetic long peptide complex can activate antigen-presenting cells such as the Toll-like receptor agonist, any of them can be widely used.

The same results as in Fig. 2 were reproduced even when the synthetic long peptide was changed from the MAGE-A4 protein-derived p264:40 to the mERK2 protein-derived p121:37 (37 aa) (Fig. 3). The result indicates that definite immune delivery by simultaneous administration of the CHP-synthetic long peptide complex and the immunopotentiating agent is not limited to the synthetic long peptides having particular sequences.

The same test as in Fig. 1 was conducted except that the CHP was replaced by CHP-NH₂, CHP-COOH or CHESG in order to investigate the effects of the size and charge of the hydrophobized polysaccharide on the vaccine performance (Fig. 4). Compounds in which a non-ionic CHP is rendered ionic by chemical modification are CHP-NH₂ and CHP-COOH (Table 1). However, after the CHP-COOH is complexed with the synthetic long peptide, its zeta potential is approximately zero and it is actually non-ionic. In the CHESG, the sugar chain moiety is replaced by glycogen to enlarge the particle size (Table 1). As a result of the test, the enhancing effects by the CpG oligo DNA were apparently attenuated in the administration group of the long peptide vaccine complexed with the CHP-NH₂ or CHESG. In the administration group of the long peptide vaccine complexed with the CHP-COOH, the enhancing effects were not remarkably attenuated by the CpG oligo DNA. This result revealed that the hydrophobized polysaccharide used in the present invention desirably meets conditions that it is non-ionic after complexation with the synthetic long peptide and the particle size is approximately less than 180 nm, preferably less than 100 nm. In relation to the conventional vaccine antigen delivery system using a liposome, there are reports that the larger the size is and the higher the charge is, the better the immune delivery by the vaccine is (Non Patent Documents 14-16). However, in the case of the hydrophobized polysaccharide of the present invention, it was shown that the smaller the size after the complexation with the long peptide is, the more effective the non-ionicity after the complexation is, and consequently it became clear that the case is completely different from the cases of the conventional liposome.

**[Table 1]**

| Hydrophobized polysaccharide | Average molecular weight | Before the complexation with the synthetic long peptide | | After the complexation with the synthetic long peptide (MAGE-A4 p264:40) | |
|---|---|---|---|---|---|
| | | Particle size, nm | Zeta potential, mV | Particle size, nm | Zeta potential, mV |
| CHP | 100,000 | 31 | N/A | 55 | 0.3 |
| CHP-NH₂ | 100,000 | 39 | 6.9 | 86 | 16.1 |
| CHP-COOH | 100,000 | 30 | -14.7 | 44 | -1.1 |
| CHESG | 2,100,000 | 115 | N/A | 183 | -1.9 |

The same test as in Fig. 1 was conducted except that the length of the long peptide was changed from 40 aa to 80 aa or 120 aa (Fig. 5). The sequence of the long peptide of 80 aa or 120 aa is two or three repeated sequences respectively of the 40 aa long peptide. Although the complexes of the long peptides of respective lengths with the CHP delivered the objective killer T cell response, the longer the amino acid length of the long peptide was, the weaker the enhancing effects by the CpG oligo DNAbecame. This result first revealed that the length of the long peptide used in the present invention is 120 aa or less, preferably 80 aa or less, more preferably 40 aa or less.

The same test as in Fig. 5 was conducted except that the long peptide was changed from the MAGE-A4 protein-derived peptide to the mERK2 protein-derived peptide (Fig. 6). Similar to Fig. 5, the longer the amino acid length of the long peptide was, the weaker the enhancing effects by the CpG oligo DNA became.

In the cancer-bearing mouse model in which mouse colon cancer cell CT 26 expressing MAGE-A4 protein was implanted, the simultaneous administration group of CHP-MAGE-A4 p264:40 complex and CpG oligo DNA showed significant tumor antiproliferative effects compared to the PBS administration group (p<0.0001), and also showed significantly excellent antiproliferative effects compared to the simultaneous administration group of MAGE-A4 p264:40 and CpG oligo DNA (p<0.05) (Fig. 7). The results considerably match the ability to deliver the antigen-specific T cells of each vaccine shown in Fig. 1.

Also in the cancer-bearing mouse model in which mouse fibrosarcoma cell CMS5a expressing mERK2 protein was implanted, the simultaneous administration group of CHP-mERK2 p121:37 complex and CpG oligo DNA showed significant tumor antiproliferative effects compared to the PBS administration group (p<0.03) (Fig. 8). Meanwhile, the simultaneous administration group of mERK2 p121:37 and CpG oligo DNA showed no significant antiproliferative effect.

These results show that the single administration of the synthetic long peptide, the simultaneous administration of the synthetic long peptide and the immunopotentiating agent and the single administration of the hydrophobized polysaccharide-synthetic long peptide complex are all insufficient to deliver the antigen-specific killer T cells and helper T cells, and cannot be expected to have therapeutic effects as vaccines for cancer treatment, but clearly show that the vaccine can receive maximum effects of the immunopotentiating agent as long as the hydrophobized polysaccharide-synthetic long peptide complex and the immunopotentiating agent are simultaneously administered, and activation of the antigen-specific T cells and expression of therapeutic effects on tumors can be achieved. Also, the results demonstrated the physicochemical properties of the hydrophobized polysaccharide and the peptide length of the long peptide required for the present invention to exert the maximum effects.

According to the embodiment, the vaccine for cancer treatment having extremely high therapeutic effects on cancers could be provided.

## Claims

1. A vaccine preparation for cancer treatment composed of: a drug which comprises a complex of one or more synthetic long peptides derived from a tumor-specific antigenic protein and/or pathogen-derived antigenic protein and simultaneously containing a CD8 positive, cytotoxic T-cell recognizing epitope and a CD4 positive, helper T-cell recognizing epitope with a hydrophobized polysaccharide; and one or more immunopotentiating agents.

2. The vaccine preparation for cancer treatment according to claim 1, wherein the synthetic long peptide is a polypeptide composed of 23-120 amino acids comprising at least two or more T-cell recognizing epitopes.

3. The vaccine preparation for cancer treatment according to claim 2, wherein when a successive sequence of 22 or less amino acids in whole amino acid sequences is used for the synthetic long peptide, immune tolerance, wasting and dysfunction can occur in the CD8-positive cytotoxic T cell.

4. The vaccine preparation for cancer treatment according to claim 1, wherein the synthetic long peptide is a polypeptide composed of 23-80 amino acids comprising at least two or more T-cell recognizing epitopes.

5. The vaccine preparation for cancer treatment according to claim 1, wherein the synthetic long peptide is a polypeptide composed of 23-40 amino acids comprising at least two or more T-cell recognizing epitopes.

6. The vaccine preparation for cancer treatment according to claim 1, wherein the CD8 positive, cytotoxic T-cell recognizing epitope is a part of an amino acid sequence of the tumor-specific antigenic protein.

7. The vaccine preparation for cancer treatment according to claim 1, wherein the CD4 positive, helper T-cell recognizing epitope is a part of the amino acid sequence of the tumor-specific antigenic protein.

8. The vaccine preparation for cancer treatment according to claim 17, wherein the tumor-specific antigenic protein is selected from MAGE family, NY-ESO-1, SPANX family, HER2, WT1, hTERT, RHAMM and survivin.

9. The vaccine preparation for cancer treatment according to claim 1, wherein the CD8-positive helper T-cell recognizing epitope is a part of an amino acid sequence of the pathogen-derived protein.

10. The vaccine preparation for cancer treatment according to claim 1, wherein the CD4 positive, helper T-cell recognizing epitope is a part of the amino acid sequence of the pathogen-derived protein.

11. The vaccine preparation for cancer treatment according to claim 1, wherein the pathogen-derived antigenic protein is selected from hepatitis B virus, hepatitis C virus, EB virus, human papillomavirus, human T-lymphotropic virus, human herpes virus 8 and human immunodeficiency virus.

12. The vaccine preparation for cancer treatment according to claim 1, wherein a polysaccharide constituting the hydrophobized polysaccharide is a pullulan or a mannan.

13. The vaccine preparation for cancer treatment according to claim 1, wherein a hydrophobic group of the hydrophobized polysaccharide is a cholesterol.

14. The vaccine preparation for cancer treatment according to claim 1, wherein the hydrophobized polysaccharide is non-ionic.

15. The vaccine preparation for cancer treatment according to claim 1, wherein a particle size after complexation of the hydrophobized polysaccharide and the synthetic long peptide is 180 nm or less.

16. The vaccine preparation for cancer treatment according to claim 1, wherein the particle size after complexation of the hydrophobized polysaccharide and the synthetic long peptide is 100 nm or less.

17. The vaccine preparation for cancer treatment according to claim 16, wherein the immunopotentiating agent is an agonist of a Toll-like receptor.

18. The vaccine preparation for cancer treatment according to claim 17, wherein the agonist of the Toll-like receptor is a poly-IC RNA or a CpG oligo DNA.

19. The vaccine preparation for cancer treatment according to claim 1, wherein the immunopotentiating agent is a chemotherapeutic agent which activates an antigen-presenting cell.

20. The vaccine preparation for cancer treatment according to claim 19, wherein the chemotherapeutic agent activating the antigen-presenting cell is a taxane-based compound or an anthracycline-based compound.

21. The vaccine preparation for cancer treatment according to claim 1, wherein the immunopotentiating agent is an agent having effects to prevent the antigen-presenting cell from gaining an immunosuppressive activity.

22. The vaccine preparation for cancer treatment according to claim 21, wherein the agent having the effects to prevent the antigen-presenting cell from gaining the immunosuppressive activity is a JAK/STAT inhibitor or an IDO inhibitor.


SEQUENCE LISTING

<110>  MIE UNIVERSITY
       TOKYO MEDICAL AND DENTAL UNIVERSITY

<120>  vaccine formulation for curing cancer

<130>  JP2012005MIU

<150>  JP2011-189234
<151>  2011-08-31

<160>  9

<170>  PatentIn version 3.5

<210>  1
<211>  40
<212>  PRT
<213>  Human

<220>
<223>  MAGE-A4 p264:40

<400>  1

Gly Ser Asn Pro Ala Arg Tyr Glu Phe Leu Trp Gly Pro Arg Ala Leu
1               5                   10                  15

Ala Glu Thr Ser Tyr Val Lys Val Leu Glu His Val Val Arg Val Asn
            20                  25                  30

Ala Arg Val Arg Ile Ala Tyr Pro
        35                  40

<210>  2
<211>  9
<212>  PRT
<213>  Human

<220>
<223>  MAGE-A4 p265

<400>  2

Ser Asn Pro Ala Arg Tyr Glu Phe Leu
1               5

<210>  3
<211>  16
<212>  PRT
<213>  Human

<400>  3

Val Lys Val Leu Glu His Val Val Arg Val Asn Ala Arg Val Arg Ile
1               5                   10                  15

<210>  4
<211>  80
<212>  PRT
<213>  Artificial

<220>
<223>  MAGE-A4 p264:80

<400>  4

Gly Ser Asn Pro Ala Arg Tyr Glu Phe Leu Trp Gly Pro Arg Ala Leu
1               5                   10                  15

Ala Glu Thr Ser Tyr Val Lys Val Leu Glu His Val Val Arg Val Asn
            20                  25                  30

Ala Arg Val Arg Ile Ala Tyr Pro Gly Ser Asn Pro Ala Arg Tyr Glu
        35                  40                  45

Phe Leu Trp Gly Pro Arg Ala Leu Ala Glu Thr Ser Tyr Val Lys Val
    50                  55                  60

Leu Glu His Val Val Arg Val Asn Ala Arg Val Arg Ile Ala Tyr Pro
65                  70                  75                  80

<210>  5
<211>  120
<212>  PRT
<213>  Artificial

<220>
<223>  MAGE-A4 p264:120

<400>  5

Gly Ser Asn Pro Ala Arg Tyr Glu Phe Leu Trp Gly Pro Arg Ala Leu
1               5                   10                  15

Ala Glu Thr Ser Tyr Val Lys Val Leu Glu His Val Val Arg Val Asn
            20                  25                  30

Ala Arg Val Arg Ile Ala Tyr Pro Gly Ser Asn Pro Ala Arg Tyr Glu
        35                  40                  45

Phe Leu Trp Gly Pro Arg Ala Leu Ala Glu Thr Ser Tyr Val Lys Val
    50                  55                  60

Leu Glu His Val Val Arg Val Asn Ala Arg Val Arg Ile Ala Tyr Pro
65                  70                  75                  80

Gly Ser Asn Pro Ala Arg Tyr Glu Phe Leu Trp Gly Pro Arg Ala Leu
                85                  90                  95

Ala Glu Thr Ser Tyr Val Lys Val Leu Glu His Val Val Arg Val Asn
            100                 105                 110

Ala Arg Val Arg Ile Ala Tyr Pro
        115                 120

<210>  6
<211>  37
<212>  PRT
<213>  Human

<220>
<223>  mERK2 p121:37

<400>  6

Asn Asp His Ile Ala Tyr Phe Leu Tyr Gln Ile Leu Arg Gly Leu Gln
1               5                   10                  15

Tyr Ile His Ser Ala Asn Val Leu His Arg Asp Leu Lys Pro Ser Asn
            20                  25                  30

Leu Leu Leu Asn Thr
        35

<210>  7
<211>  9
<212>  PRT
<213>  Human

<220>
<223>  9m

<400>  7

Gln Tyr Ile His Ser Ala Asn Val Leu
1               5

<210>  8
<211>  17
<212>  PRT
<213>  Human

<400>  8

Arg Gly Leu Gln Tyr Ile His Ser Ala Asn Val Leu His Arg Asp Leu
1               5                   10                  15

Lys

<210>  9
<211>  74
<212>  PRT

<213>  Artificial

<220>
<223>  mERK2 p121:74

<400>  9

Asn Asp His Ile Ala Tyr Phe Leu Tyr Gln Ile Leu Arg Gly Leu Gln
1               5                   10                  15

Tyr Ile His Ser Ala Asn Val Leu His Arg Asp Leu Lys Pro Ser Asn
            20                  25                  30

Leu Leu Leu Asn Thr Asn Asp His Ile Ala Tyr Phe Leu Tyr Gln Ile
        35                  40                  45

Leu Arg Gly Leu Gln Tyr Ile His Ser Ala Asn Val Leu His Arg Asp
    50                  55                  60

Leu Lys Pro Ser Asn Leu Leu Leu Asn Thr
65                  70